# EUROPEAN PATENT SPECIFICATION

(11) **EP 1 351 636 B1**
(45) Date of publication and mention of the grant of the patent: **19.04.2006**
(21) Application number: 01271191.7
(22) Date of filing: 19.12.2001
(51) Int. Cl.: A61F 13/64

(54) **ABSORBENT ARTICLE**
ABSORBIERENDER ARTIKEL
ARTICLE ABSORBANT

(30) Priority: 20.12.2000 SE 0004759
(43) Date of publication of application: 15.10.2003
(73) Proprietor: SCA Hygiene Products AB, 405 03 Göteborg (SE)
(72) Inventor: HANSSON, Roy, S-431 50 Mölndal (SE)
(74) Representative: Egeröd, Lisbeth
(86) International application number: PCT/SE2001/002843
(87) International publication number: WO 2002/049568

(56) References cited:
- EP-A2- 0 528 282
- WO-A1-01/21126
- WO-A1-99/22688
- FR-A1- 2 586 558
- SE-C2- 504 624
- US-A- 4 662 875
- US-A- 5 897 546

## Description

The present invention relates to an absorbent article such as a diaper and an incontinence guard comprising a liquid permeable topsheet, a liquid impermeable backsheet and an absorbent body enclosed therebetween, wherein the article having a front portion, a rear portion and a crotch portion therebetween, and further is provided with a pair of belt portions attached to the rear portion of the article and which are intended to be fastened together around the waist of the wearer and wherein one belt portion is provided with first fastening means intended to be attached against the other belt portion and where said front portion is provided with attachment means intended to be attached to the belt portions, in such a way that the article will assume a pantlike shape, where the belt portions form a part of the waist portions of the pant.

### Background of the invention

Diapers and incontinence guards usually exhibit a garment portion holding an absorbent body in place against the user's body and attachment means, which hold the garment portion in place also when the user is moving. A common type of attachment means are adhesive tapes or hook and loop fasteners of the touch-and-close type, which directly attach the front and rear portions of the absorbent article to each other. It is further known, through e.g., EP-A-0 287 388, EP-A-0 409 307, EP-A-0 528 282, EP-A-0 605 012 and FR-A-2 586 558, to attach the front and rear portions of the article by means of a belt, wherein the possibilities to adjust the fit are improved. The belt also lead to a simplified change of the diaper and incontinence guard, respectively, in particular on a standing person.

A conventional diaper for children is in general applied having the child in a lying position. The attachment means are usually arranged on the rear portion and are attached to the front portion. This kind of application often requires the aid from another person. However, for an adult user it is more desirable to self be able to apply the incontinence guard. On a common type of belt diaper, the belt portions are therefore first attached around the waist. When the incontinence guard is fixed around the waist in this way, the user may reach after the rest of the incontinence guard between the legs and then the crotch portion of the incontinence guard is applied in the correct position by fastening the front portion of the diaper to the outside of the belt portions using hook and loop fasteners or tape tabs being arranged on the front portion and/or the belt portion. This design makes also possible for nursing personnel to apply the diaper on a standing person or for the user to apply the diaper on himself/herself in a standing position.

One problem for the user or the nursing personnel is to know whether the absorbing diaper part of the article is centred on the back of the user. When the belt is applied, i.e., when the belt portions is connected to each other on the belly of the wearer, it is difficult to know in which way the diaper part of the article is fit on the user, since one can not visually see how the article fits on the back. When the front portion is attached against the belt portions, it can easily happen that the article, if it has ended up wrong around the wearer, not provides the guard it is intended to provide and maybe cause discomfort to the wearer.

Therefore there is a need for absorbent article provided with a belt, wherein the belt adapts itself to the user using the diaper and also feels comfortable to wear.

### Summary of the invention

The object of the present invention is to provide a belted diaper or incontinence guard being comfortable to wear and which fits persons having different sizes. This object is being solved in that each belt portion is provided with at least one indicium, being placed on a appropriate distance from the attachment of each belt portion to the rear portion of the article, whereby each indicium on one belt portion has its corresponding indicium on the opposite belt portion.

### Brief description of drawings

The invention will in the following be closer described with reference to an embodiment shown in the accompanying drawing.

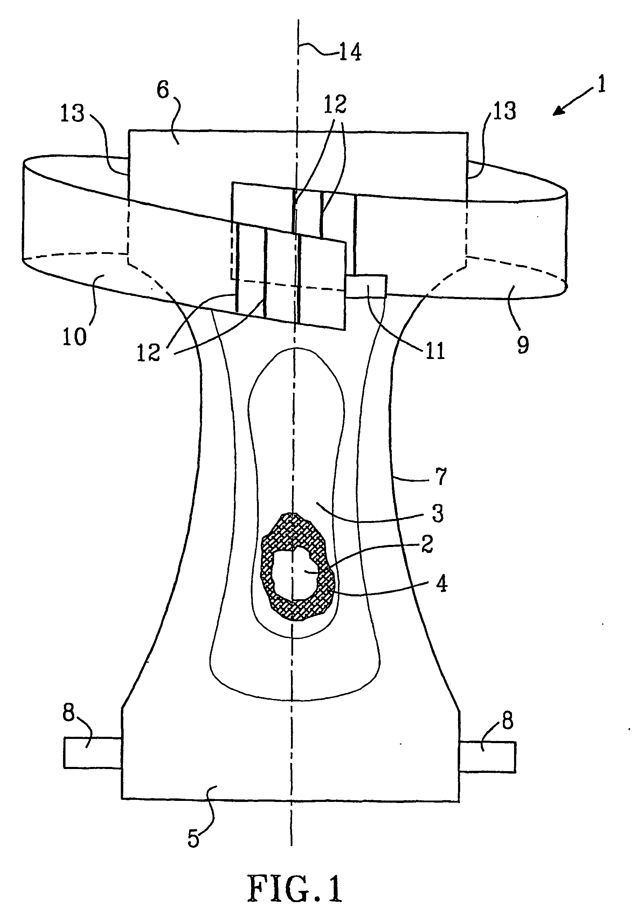

Fig. 1 schematically shows a perspective view of a diaper or incontinence guard according to the invention.

### Detailed description of the invention

The drawing shows an embodiment of a diaper or incontinence guard 1 comprising a liquid impermeable backsheet 2, a liquid permeable topsheet 3 and an absorbent body 4 enclosed therebetween. The liquid permeable topsheet 3 can consist of a nonwoven material, e.g., a spunbond material of continuous filaments, a meltblown material, a bonded carded fibrous web or a perforated plastic film. The liquid impermeable backsheet 2 may consist of a plastic film, a nonwoven material coated with a liquid impervious material or a hydrophobic nonwoven material, which resists liquid penetration. The liquid impermeable backsheet 2 may also be a vapour permeable material.

The topsheet 3 and the backsheet material 2 have a somewhat greater extension in the plane than the absorbent body 4 and extend outside the edges thereof. The layers 2 and 3 are connected to each other within the projecting portions thereof, e.g., by gluing or welding by heat or ultrasonic.

The absorbent body 4 can be of any conventional kind. Examples of commonly occurring absorbent materials are cellulose fluff pulp, tissue layers, highly absorbent polymers (so called superabsorbents), absorbent foam materials, absorbent nonwoven materials or the like. It is common to combine cellulose fluff pulp with superabsorbents in an absorbent body. It is also common to have absorbent bodies comprising layers of different material with different properties with respect to liquid acquisition capacity, liquid distribution capacity and storage capacity. It is well known to the person skilled in the art and does therefore not have to be described in detail. The thin absorbent bodies, which are common in for example baby diapers and incontinence guards often, comprise a compressed mixed or layered structure of cellulose fluff pulp and superabsorbent.

The diaper/incontinence guard is intended to enclose the lower part of the wearer's trunk like a pair of absorbent pants. It comprises a front portion 5, intended during use to be worn on the front part of the user's body, a rear portion 6, intended during use to be worn on the rear part of the user's body, and a crotch portion 7 located between the front and rear portions and which is intended to be worn in the crotch part of the user between the legs. The front portion 5 exhibits a pair of tape tabs 8 or another kind of fastening means such as hook and loop fasteners.

A pair of belt portions 9,10 are with one end attached, e.g., glued or ultrasonically welded, to the rear portion 6 of the diaper. The belt portions 9, 10 are with their opposite ends intended to be fastened together by means of first fastening means 11, e.g. a hook-and-loop type fastener or tape tabs, intended to be attached against the outsides of the opposite belt portion. The second fastening means 8 of the front portion 5, such as for instance hook and loop fasteners or tape tabs, is intended to be attached against the outsides of the belt portions 9, 10 in order to fasten together the diaper/incontinence guard to the desired pantlike shape.

The width of the belt portions 9, 10 should be between 3-20 cm, preferably between 7-15 cm. The belt portions 9, 10 comprise according to an embodiment a laminate, wherein a carrier material forms the outside of the belt and a soft nonwoven forms the inside of the belt, intended to be in direct contact with the skin of the user. A suitable nonwoven material can be a spunbond material of e.g., polypropylene or polyethylene fibres. Conjugate fibres may also be used. Another suitable nonwoven material can be a carded thermobonded material of e.g., polypropylene, polyester or conjugate fibres. A plastic film or another suitable material e.g., nonwoven, may be used as carrier material. The carrier material should be adapted to function as a reception surface for both the attachment means 8 and 11, wherein in the case where these are tape tabs, a plastic film is suitable. In the case wherein other kinds of fastenings means is used instead of tape tabs, e.g., a hook and loop type fastener, another kind of carrier material is used, in particular a nonwoven material, which can act as a reception surface for the fastening means in question. Separate reception surfaces for fastening means could also be arranged on the outsides of the belt.

The belt portions 9, 10 are each provided with at least one indicium 12. The indicia 12 on the opposite belt portions are symmetrically placed in the longitudinal direction of the belt portions 9, 10, on a certain distance from the attachment 13 of each belt portion 9, 10 to the rear portion 6 of the diaper/incontinence guard. Thus, each indicium 12 on one belt portion 9 has its corresponding indicium on the opposite belt portion 10, placed on essentially the same distance from the attachment 13 of each belt portion 9,10 to the rear portion 6 of the article. Upon application of the article the belt portions 9,10 are first attached around the waist of the wearer and said indicia 12 on the different belt portions 12 are fitted against each other and are levelled against the navel on the user. Thereby is ensured that the centre line 14 of the article is located at the centre of the back of the user, since the position of the navel gives the centre position on the front side of the user. Thus, the share of the belt portion 9 extending in one direction from the attachment 13 of the belt portion 9 to the rear portion 6 around the patient is equally large as the share of the belt portion 10 extending in the other direction around the patient.

For reasons of manufacturing, the indicia 12 are preferably located on the inside portion of the belt portion, but the indicia may be located on both sides or on the outsides of the belt portions. Preferably, there are two or more indicia 12 on each belt portion 9,10, so that an adaptation of the circumference of the belt to the size of the user can be performed. Said indicia 12 can in pairs consist of stripes, lines, dots, symbols, letters, ornamental designs, images, etc. having the same or different colour. Said indicia 12 can consist of the same pattern but in pairs have different colours.

The materials used in the present invention may be of a vapour permeable material, especially for providing a breathable belt. The following experiments are examples ofbelt materials that can be used in the belt portions according to the invention.

### Example 1

### Air permeability test

Four materials intended for use in the belt portions were tested using the EDANA 140.1-99 using the Textest FX 330 equipment. The materials were tested at eight different pressures and the permeability for air was measured. The results are presented in Table 1 below.

The materials being tested were:
1 F43, a nonwoven carrier material of polypropylene (Freudenberg) having a thread of polyamide arranged in the nonwoven material in order to provide a loop material. Said material having a basis weight of 43 g/m³ (nonwoven 30 + thread 13 = 43 g/m³)
2 F53, a nonwoven carrier material of polypropylene (Freudenberg) having a thread of polyamide arranged in the nonwoven material in order to provide a loop material. Said material having a basis weight of 53 g/m³ (nonwoven 40 + thread 13 = 53 g/m³)
3 F73, a nonwoven carrier material of polypropylene (Freudenberg) having a thread of polyamide arranged in the nonwoven material in order to provide a loop material. Said material having a basis weight of 73 g/m³ (nonwoven 60 + thread 13 = 73 g/m³)
4 A nonwoven material (SCA) for use in a belt having 3 layers of nonwoven material of polypropylene. It comprises of one layer intended to be the outside layer of the belt of carded nonwoven having a basis weight of 30 g/m³, a spunbond material having a basis weight of 40 g/m³ in between and a layer of a carded nonwoven having a basis weight of 22 g/m³ intended to be faced against the skin of the user.

From the table 1 below it is apparent that a thinner material has a larger permeability for air.

**Table 1**

| | **Air permeability** | | | | | | | |
|---|---|---|---|---|---|---|---|---|
| | **Material 1** | | **Material 2** | | **Material 3** | | **Material 4** | |
| Pressure (Pa) | m³/m²/min | STD | m³/m²/min | STD | m³/m²/min | STD | m³/m²/min | STD |
| 75 | 81 | 8 | 59 | 4 | 34 | 3 | 21 | 1 |
| 150 | 136 | 9 | 106 | 7 | 67 | 8 | 41 | 3 |
| 198 | 173 | 11 | 127 | 5 | 82 | 7 | 52 | 3 |
| 300 | 238 | 16 | 184 | 14 | 111 | 9 | 72 | 6 |
| 400 | 271 | 11 | 229 | 10 | 143 | 19 | 92 | 4 |
| 500 | 326 | 14 | 267 | 15 | 169 | 15 | 112 | 9 |
| 750 | 441 | 28 | 344 | 23 | 237 | 17 | 154 | 9 |
| 1000 | 531 | 33 | 416 | 30 | 279 | 25 | 198 | 7 |

### Example 2

### Investigation of breathable belt materials

The same four materials as in example 1 were tested for the water vapour permeability using the method ASTM E96-95 (in a climate of 38°C and 90 % relative humidity). Each material was tested six times. The results are presented in Table 2 below. It is evident from the table that material number 4 exhibits the highest breathability measured as the permeability for water vapour expressed in grams water vapour per area unit per 24 hours of the tested material. A well performing breathable material usually exhibits values exceeding 2000 g/m²/24 h.

**Table 2**

| **Permeability for water vapour** | | | | | | | |
|---|---|---|---|---|---|---|---|
| **Material 1** | | **Material 2** | | **Material 3** | | **Material 4** | |
| g/m²/24 h | STD | g/m²/24 h | STD | g/m²/24 h | STD | g/m²/24 h | STD |
| 3603 | 114 | 3529 | 0 | 3603 | 114 | 3755 | 149 |

The invention is of course not limited to the above described and on the drawing showed embodiments, but can be modified within the scope of the claims.

## Claims

1. Absorbent article such as a diaper and an incontinence guard comprising a liquid permeable topsheet (3), a liquid impermeable backsheet (2) and an absorbent body (4) enclosed therebetween, said article having a front portion (5), a rear portion (6) and a crotch portion (7) therebetween, and further is provided with a pair of belt portions (9, 10) attached to the rear portion (6) of the article and which are intended to be fastened together around the waist of the wearer, wherein the one belt portion (10) carries first fastening means (11) intended to be attached against the opposite belt portion (9) and where said front portion (5) exhibits second fastening means (8) intended to be attached to the belt portions (9, 10), in such a way that the article will assume a pantlike shape, where the belt portions (9, 10) form a part of the waist portions of the pant,
**characterised in,**
**that** each belt portions (9,10) are provided with at least one indicium (12), being placed on a appropriate distance from the attachment (13) of each belt portion (9, 10) to the rear portion (6) of the article, whereby each indicium (12) on one belt portion (9) has its corresponding indicium on the opposite belt portion (10).

2. Absorbent article according to claim 1,
**characterised in,**
**that** said indicia (12) are an ornamental design.

3. Absorbent article according to claim 1,
**characterised in,**
**that** said indicia (12) are symbols.

4. Absorbent article according to claim 1,
**characterised in,**
**that** said indicia (12) are coloured indicia.

## Patentansprüche

1. Absorbierender Gegenstand, wie beispielsweise eine Windel und einen Inkontinenzschutz umfassend eine flüssigkeitsdurchlässige Oberlage (3), eine flüssigkeitsundurchlässige Decklage (2) und einen dazwischen eingeschlossenen Absorptionskörper (4), wobei der Gegenstand einen Vorderabschnitt (5), einen Hinterabschnitt (6) und einen Schrittabschnitt (7) dazwischen aufweist, und ferner mit zwei Gürtelabschnitten (9, 10) versehen ist, die an dem Hinterabschnitt (6) des Gegenstands angebracht sind und dazu gedacht sind, um die Taille des Trägers befestigt zu werden, wobei der eine Gürtelabschnitt (10) eine erste Verschlusseinrichtung (11) trägt, die dazu gedacht ist, an dem entgegengesetzten Gürtelabschnitt (9) angebracht zu werden und wobei der Vorderabschnitt (5) eine zweite Verschlusseinrichtung (8) aufweist, die dazu gedacht ist, derart an dem Gürtelabschnitt (9, 10) angebracht zu werden, dass der Gegenstand eine höschenähnliche Form annehmen wird, wobei die Gürtelabschnitte (9, 10) einen Teil des Taillenabschnitt des Höschens bilden,
**dadurch gekennzeichnet, dass**
jeder der Gürtelabschnitte (9, 10) mit wenigstens einem Vermerk (12) versehen ist, der in einem geeigneten Abstand von der Befestigung (13) jedes Gürtelabschnitts (9, 10) am Hinterabschnitt (6) des Gegenstands angeordnet ist, wobei jeder Vermerk (12) auf einem Gürtelabschnitt (9) seinen entsprechenden Vermerk auf dem entgegengesetzten Gürtelabschnitt (10) aufweist.

2. Absorbierender Gegenstand nach Anspruch 1,
**dadurch gekennzeichnet, dass**
die Vermerke (12) eine dekorative Ausgestaltung aufweisen.

3. Absorbierender Gegenstand nach Anspruch 1,
**dadurch gekennzeichnet, dass**
die Vermerke (12) Symbole sind.

4. Absorbierender Gegenstand nach Anspruch 1,
**dadurch gekennzeichnet, dass**
die Vermerke (12) gefärbte Vermerke sind.

## Revendications

1. Article absorbant tel qu'une couche-culotte ou une protection contre l'incontinence comprenant une feuille avant (3) perméable aux liquides, une feuille arrière (2) imperméable aux liquides et un corps absorbant (4) enfermé entre elles, ledit article ayant une partie avant (5), une partie arrière (6) et une partie entrejambe (7) entre elles, et est pourvu en outre d'une paire de parties de ceinture (9, 10) fixées à la partie arrière (6) de l'article et qui sont destinées à être attachées l'une à l'autre autour de la taille de l'utilisateur, dans lequel une partie de ceinture (10) porte un premier moyen de fermeture (11) destiné à être fixé contre la partie de ceinture opposée (9) et où ladite partie avant (5) présente un deuxième moyen de fermeture (8) destiné à être fixé aux parties de ceinture (9, 10), de telle manière que l'article va prendre une forme de culotte, où les parties de ceinture (9, 10) forment une partie des parties de taille de la culotte,
**caractérisé en ce que** chaque partie de ceinture (9, 10) est munie d'au moins un repère (12) placé à une distance appropriée de la fixation (13) de chaque partie de ceinture (9, 10) sur la partie arrière (6) de l'article, grâce à quoi chaque repère (12) d'une partie de ceinture (9) a son repère correspondant sur la partie de ceinture opposée (10).

2. Article absorbant selon la revendication 1, **caractérisé en ce que** lesdits repères (12) sont des dessins ornementaux.

3. Article absorbant selon la revendication 1, **caractérisé en ce que** lesdits repères (12) sont des symboles.

4. Article absorbant selon la revendication 1, **caractérisé en ce que** lesdits repères (12) sont des repères en couleur.
